# EUROPEAN PATENT APPLICATION

(11) **EP 4 510 055 A1**
(43) Date of publication of application: **19.02.2025**
(21) Application number: 23191221.3
(22) Date of filing: 12.08.2023
(51) Int. Cl.: G06Q 10/0631, G16H 10/60, G16H 40/20

(54) **MEDICAL OPERATIONS SUPPORT SYSTEM**

(71) Applicant: Yolo GmbH, 42113 Wuppertal (DE)
(72) Inventor: Dib, Ribal, 42113 Wuppertal (DE)
(74) Representative: noventive Patentanwaltsgesellschaft mbH

(57) **Abstract**

A medical operations support system (10), comprises two or more of the following devices: a patient interaction device (700), a patient data storage device (16) for storing patient data, configured to store and/or retrieve data upon request from another device of the medical operations support system (10), a diagnosis and treatment device (400), an invoice creation device (100), an appointment scheduling device (200), an alert creation device (300), a staff management device (500), a warehouse management device (600).

## Description

The invention relates to a medical operations support system.

Systems for supporting organisations are well known in the art. It is for example known to allow clients access to electronic calendars of staff to schedule appointments. It is also known to keep timesheets electronically and derive invoices from said timesheets.

Medical operations present a number of hard to coordinate tasks such as staff and warehouse management, appointment scheduling and information distribution. The coordination of these tasks is made more difficult by constraints imposed by, for example, treatment regimen which only allow for a limited rescheduling of appointments, the availability of staff and materials. Furthermore, many work hours are spent on clerical tasks such as creation, verification and issue of invoices. Complicated billing rules of insurance companies make this task even harder.

In light of the above, the invention solves the problem of efficiently coordinating clerical tasks in a medical practice.

The problem is solved by means of a device according to claim 1 as well as a computer readable medium according to claim 7. Further advantageous embodiments are the object of the dependent claims.

To solve the problem, a medical operations support system comprises two or more of the following devices: a patient interaction device, a patient data storage device for storing patient data, configured to store and/or retrieve data upon request from another device of the medical operations support system, a diagnosis and treatment device, an invoice creation device, an appointment scheduling device, an alert creation device, a staff management device, a warehouse management device.

Each of the support devices is able to automate an aspect of medical operations, making it more efficient, more precise, allow automated learning from past events and faster decision-making.

In some embodiments, in which the medical operations support system comprises the invoice creation device, wherein the invoice creation device comprises a medical code determination unit configured to determine one or more medical diagnosis codes and/or medical procedure codes from one or more medical notes.

Automated analysis of medical notes saves time, increases invoicing accuracy and optimizes revenue and costs.

In some embodiments, the medical code determination unit comprises an Al module comprising an encoder module configured to convert medical notes to a hidden representation and a decoder module for converting the hidden representation to a medical diagnosis code and/or a medical procedure code.

An Al module is, in particular, field-trainable and is able to recognize complex patterns in patient data.

In some embodiments, the invoice creation device comprises a code interdependency repository configured to guide the encoder module and/or the decoder module by providing information about which medical diagnosis codes and/or medical procedure codes may coexist and/or which medical diagnosis codes and/or medical procedure codes exclude each other.

This improves quality of the medical diagnosis and/or procedure codes determined by the invoice creation device.

In some embodiments, any of the diagnosis and treatment device, the invoice creation device, the appointment scheduling device, the alert creation device, the staff management device and the warehouse management device may comprise its proper AI module or make use of a central AI module.

Separate AI modules provide the advantage of being more easily adaptable to their individual tasks. A central AI module has the advantage, that it uses less resources than individual, smaller AI modules.

In some embodiments, the invoice creation device may comprise a text standardization module configured to receive a text of a medical note and to convert the text of the medical note into a standardized text, in particular according to a set of text formation rules and/or by means of an AI module.

Such standardized text is easier to read and comprehend by users and standardization may avoid misunderstandings, and unclear instructions.

The problem is further solved by computer readable instructions that when executed by one or more computers cause the computers to form a medical operations support system according to any of the previous claims or one or more of the patient interaction device, the patient data storage device, the diagnosis and treatment device, the invoice creation device, the appointment scheduling device, the alert creation device, the staff management device and/or the warehouse management device.

Such instructions may be stored on a transitory or non-transitory computer readable medium or may be downloadable.

Further features and variants of the invention will be apparent to the skilled person, in particular from the enclosed drawings which show example embodiments of the invention. The figures show:
- Fig. 1: a schematic overview of a medical operations support system according to an embodiment of the invention;
- Fig. 2: a schematic overview of an invoice creation device according to an embodiment of the invention;
- Fig. 3: a schematic view of a medical code determination unit;
- Fig. 4: a schematic view of an appointment scheduling device;
- Fig. 5: a schematic view of an alert creation device;

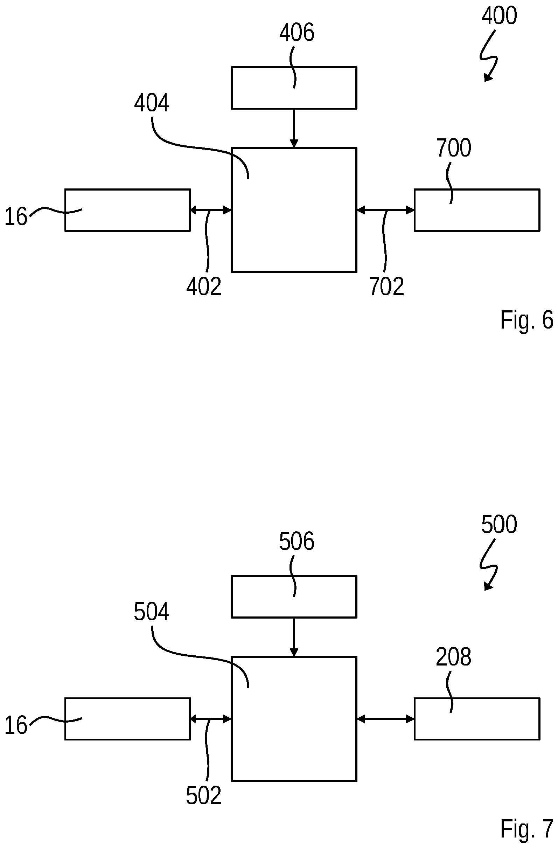

- Fig. 6: a schematic view of a diagnosis and treatment device;
- Fig. 7: a schematic view of a staff management device and

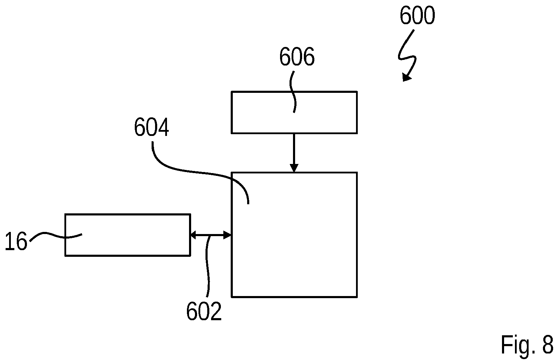

- Fig. 8: a schematic view of a warehouse management device.

The figures contain partially simplified, schematic representations. In part, identical reference signs are used for similar, but possibly not identical elements. Different views of identical elements could be scaled differently. Directional indications such as "left", "right", "top", "up" and "bottom" are to be understood with reference to the respective figure and may vary in the individual representations with respect to the object depicted.

Furthermore, elements are not necessarily shown to scale relative to one another, in particular to simplify and clearly illustrate their functional relationship. Insofar as process steps or elements are provided with enumerative designations such as, for example, "first", "second", "third" or "further", these designations, insofar as nothing else is explicitly disclosed, serve merely to differentiate and do not imply any sequence or hierarchy.

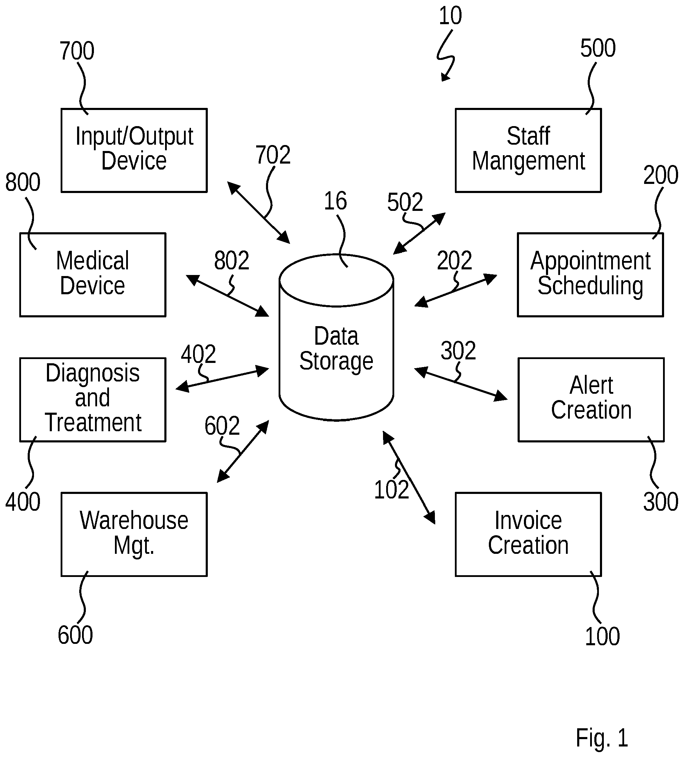

A medical operations support system 10 as shown in Fig. 1 comprises an invoice creation device 100, an appointment scheduling device 200, an alert creation device 300, a diagnosis and treatment device 400, a staff management device 500 and a warehouse management device 600. Furthermore, the medical operations support system comprises a data storage device 16.

The invoice creation device 100, the appointment scheduling device 200, the alert creation device 300, the diagnosis and treatment device 400, the staff management device 500 and the warehouse management device 600 each are connected to the data storage unit 16 by means of an invoice creation data interface 102, an appointment scheduling data interface 202, an alert creation data interface 302, a diagnosis and treatment data interface 402, a staff management data interface 502 and a warehouse management data interface 602. Furthermore, the medical operations support system 10 provides an input/output data interface 702 for connecting an input/output device 700 for interaction with operators. The medical operations support system 10 provides a medical data interface 802 for connecting a medical device 800.

Further data interfaces may be provided for direct communication between the invoice creation device 100, the appointment scheduling device 200, the alert creation device 300, the diagnosis and treatment device 400, the staff management device 500, the warehouse management device 600, the input/output device 700 and/or the medical device 800.

### Invoice Creation Device 100

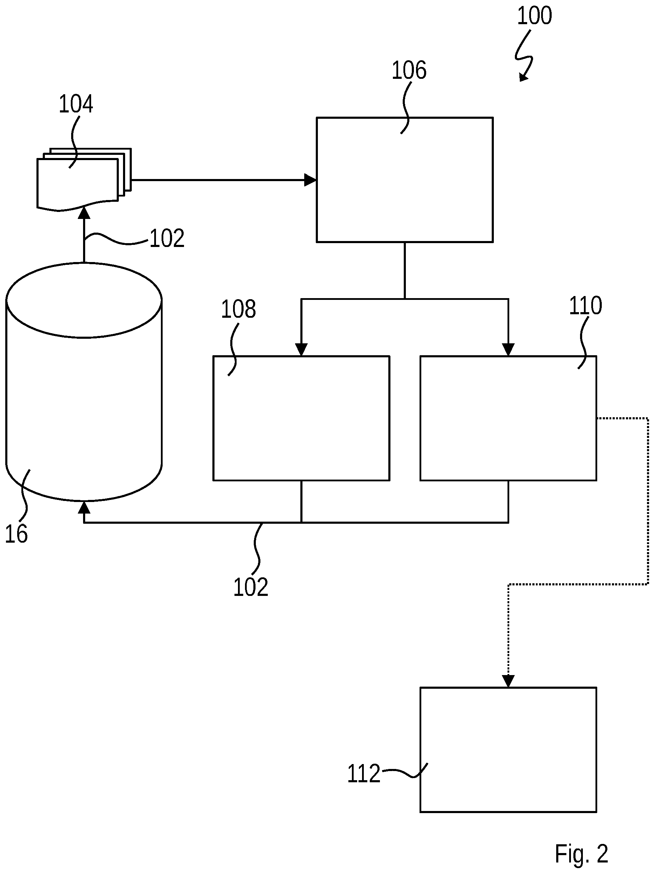

A general outline of the invoice creation device 100 is presented in **Fig. 2****.** The invoice creation device 100 comprises a medical code determination unit 106. The medical code determination unit 106 is configured to analyze one or more medical notes 104 in an analysis step. The medical code determination unit 106 is furthermore configured to determine, from results of the analysis performed in the analysis step, details of the medical proceedings described in the medical notes 104. In particular, the medical code determination unit 106 is configured to determine medical diagnosis codes 108 and medical procedure codes 110.

Medical diagnosis codes 108 comprise codes describing, for example, illnesses, types of injury and/or symptoms of a patient. From analysis of the medical notes 104 relating to one particular patient, one or multiple medical diagnosis codes 108 related to said patient may be derived.

The medical diagnosis codes 108 may conform to a national or international standard. The medical diagnosis codes 108 may, for example, conform to any revision of the International Statistical Classification of Diseases and Related Health Problems, abbreviated as ICD, published by the World Health Organization (WHO) as well as to modified variations of the ICD such as those published by national or other organizations. Examples of frequently used ICD revisions include ICD-9, ICD-10 and ICD-11 published by the WHO and ICD-9-CM and ICD-10-CM published by the United States National Center for Health Statistics (NCHS). Further national modifications are in use. Further to ICD codes, other national and/or organizational standards and/or conventions exist for describing medical diagnoses.

In some embodiments, the medical diagnosis codes 108 may comprise a representation internal to the invoice creation device 100 and/or the medical operations support system 10.

Medical procedure codes 110 comprise codes describing actions taken or to be taken during medical treatment, for example, diagnostic procedures, treatment procedures, test procedures, operations, prescriptions, therapeutic measures and/or supplementary measures. From analysis of the medical notes 104 relating to one particular patient, one or multiple medical diagnosis codes 110 related to said patient may be derived. The medical procedure codes 110 may, for example, conform to any revision of the ICD as mentioned above. Other definitions of medical procedure codes 110 may be in use such as the ICPM (International Classification of Procedures in Medicine) or the OPS (Operationen- und Prozedurenschlüssel, Germany).

In some embodiments, the medical procedure codes 110 may comprise a representation internal to the invoice creation device 100 and/or the medical operations support system 10.

The medical code determination unit 106 may receive the medical notes 104 from the data storage device 16 via the invoice creation data interface 102. The medical code determination unit 106 may furthermore store the medical diagnosis codes 108 and/or the medical procedure codes 110 in the data storage device 16.

The invoice creation device 100 may further comprise an invoice coding unit 112 which is connected to receive the medical procedure codes 110. Using the medical procedure codes 110, the invoice coding unit 112 determines which invoice codes are associated with the medical procedure codes 110. Invoice codes are codes that are used to bill medical services, in particular when the bill is to be settled by an insurance. The invoice codes may be used for digital billing and allow the insurance to check plausibility of an invoice automatically.

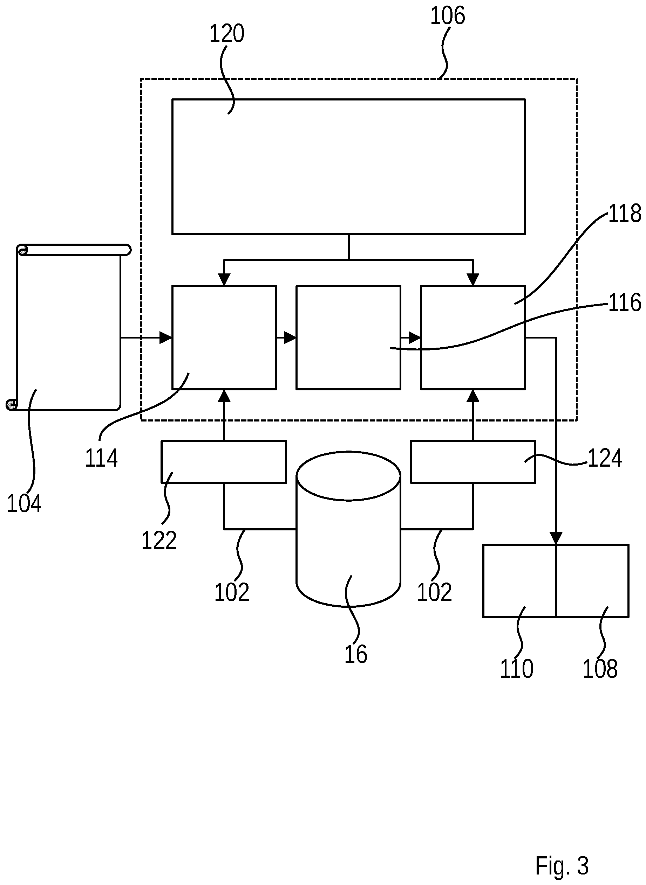

Further details relating to the medical code determination unit 106 are shown in Fig. 3. The medical code determination unit 106 comprises an encoder module 114 and a decoder module 118. The encoder module 114 and the decoder module 118 are connected to a code interdependency repository 120. Furthermore, the encoder module 114 and decoder module 118 may, for example via the interface 102, access encoder hint data 122 and/or decoder hint data 124 which are stored, for example, in the storage device 16.

The encoder module 114 is configured to extract relevant features from input text contained in the medical note 104, transforming the input text of the medical note 104 into a hidden representation 116. The hidden representation 116 may, in particular, comprise a hidden representation vector.

In some embodiments, the encoder module 114 may comprise an artificial intelligence component, for example a recurrent neural network (RNN), for example a long short-term memory (LSTM) network or a gated recurrent unit (GRU) network. The encoder module 114 is configured to separate the input text into tokens and generate the vector representation. To this end, the encoder module 114 may comprise an input embedding module. The encoder module 114 may, in particular, use any of the well-known encoders in the art, such as a one-hot encoder, a bag-of-words encoder or a tf-idf encoder.

The decoder module 118 is configured to transform the hidden representations 116 of the encoder module 114 into medical codes 108, 110. To this end, the decoder module 118 may comprise an artificial intelligence component such as, for example, an AI module or a dedicated hardware AI device.

To support the encoder module 114 and the decoder module 118, the code interdependency repository 120 comprises data on the medical coding that shall be used to output the medical codes 108, 110. The code interdependency repository 120 may, in particular, comprise data on every medical code 108, 110 permitted by the medical coding as well as a description of the medical code 108, 110 and, if the medical coding uses hierarchical medical codes 108, 110, data describing the hierarchy of the medical codes 108, 110. Furthermore, the code interdependency repository 120 may comprise data relating to how the medical codes 108, 110 are interdependent, meaning how they are related to each other. There may, for example, be a strong relation between particular medical diagnosis codes 108 and medical procedure codes 110.

The code interdependency repository 120 may, furthermore, be configured to carry out a verification step on the codes 108, 110 to verify that certain medical codes 108 and treatment codes 110 are allowed result combinations of the decoder module 118. In particular, the verification step is configured to disallow combinations of multiple medical diagnosis codes 108 and/or medical treatment codes 110 that would be harmful to patient health to be output. To this end, the code interdependency repository 120 may comprise data relating to medical diagnosis codes 108 and/or medical procedure codes 110 which may not occur together as a result of the decoder module 118.

The code interdependency repository 120 may be comprised in the invoice creation device 100. In some embodiments, the code interdependency repository 120 may be, for example, stored in the data storage device 16 and may be accessed via the invoice creation data interface 102.

The encoder module 114 may be provided with encoder context data 122 to supplement the content of the medical note 104. For example, if a medical note 104 only refers to some particular treatment, the encoder context data 122 may comprise information about the medical context in which the treatment is given, for example a medical history of the patient. The encoder context data 122 thus serves to improve the accuracy of the encoder module 114.

The decoder module 118 may also be provided with decoder context data 124. The decoder context data 124 comprises information that may not have been included in the medical note 104 and which may serve to determine the appropriate medical codes 108, 110. For example the decoder context data 124 may comprise information about the patient's insurance, in particular which medical treatment codes 110 said insurance will accept in the context of the medical diagnosis codes 108 that have been determined.

Encoder context data 122 and/or decoder context data 124 may comprise auxiliary information, in particular additional data that can be used to improve the accuracy of medical coding predictions.

In some embodiments, multiple encoder modules 114 and decoder modules 118 may be fused to form a transformer module.

The medical determination unit 106 may be configured, in particular by means of the context data 122, 124, to take into account insurance coverage, regional pricing variations, compliance with medical coding standards, previous diagnoses and treatments and/or regulations compliance.

The invoice creation device 100 may make use of artificial intelligence components, for example an AI module, an AI software module and/or an AI hardware module Such an artificial intelligence component may, for example, be comprised in the medical code determination unit 106. The use of such artificial intelligence components allows for automated medical billing and coding processes, ensures accuracy and compliance with medical coding standards, reduces billing errors and streamlines revenue cycle management. It also reduces the time and effort required to create, review and update invoices such that the staff can concentrate on essential tasks such as patient care and clinical operations. Since the invoice creation device 100 can be configured to take into account different insurances, it can help maximise revenue for its operator by ensuring that all billable services are appropriately documented and billed. At the same time, it can help minimise costs for insurance companies by providing them with accurate and detailed information enabling them to process claims more efficiently and avoid overpayments.

The invoice creation device 100 may, in some embodiments, be computer-implemented, for example by means of a computer, comprising at least one processor, volatile storage and/or non-volatile storage. To implement the invoice creation device 100, the implementation may comprise any of the following method steps:
Retrieve a medical note 104 to be analyzed from the data storage device 16.

Analyze one or more medical notes 104, to obtain one or more medical diagnosis codes 108 and/or medical treatment codes 110.

If the medical note 104 comprises an audio file, carry out a speech recognition step to obtain a medical note text.

If the medical note 104 comprises non-text data, such as lab result data, measurement data and/or measurement time series data, carry out a conversion step to convert the data into a representation suitable as input for the medical code determination unit 106. This conversion step may comprise converting the non-text data into a human-readable text.

If the medical note 104 comprises non-text data, such as imaging data, for example from CT or MRT diagnostic equipment, carry out a conversion step to convert the data into a representation suitable as input for the medical code determination unit 106. This conversion step may comprise carrying out an AI image recognition step, a measurement step to determine measurements of features represented in the imaging data and/or a feature detection step to detect medically relevant features in the imaging data.

Encode, tokenize and/or extract medically relevant textual features from the text or the textual representation obtained in one of the other method steps to obtain the hidden representation of the medical note 104. This method step may be carried out by or implemented as the encoder module 114.

Decode and/or transform the hidden representation into one or more medical diagnosis code 108 and/or medical treatment code 110.

Verify that the combination of one or more medical diagnosis code 108 and/or the medical treatment code 110 and/or other patient data is not disallowed, for example, according to the code interdependency repository 120, the encoder context data 122 and/or the decoder context data 122.

The increase in accuracy and compliance with regulations, medical coding standards and insurance policies may reduce the risk of billing-related audits, disputes and penalties. Furthermore, patient experience is improved by accurately and timely billing for services. Furthermore, accurate and up-to-date financial data may be provided, which can be used for budgeting, forecasting and identifying opportunities for cost savings or revenue growth.

### Appointment Scheduling Device 200

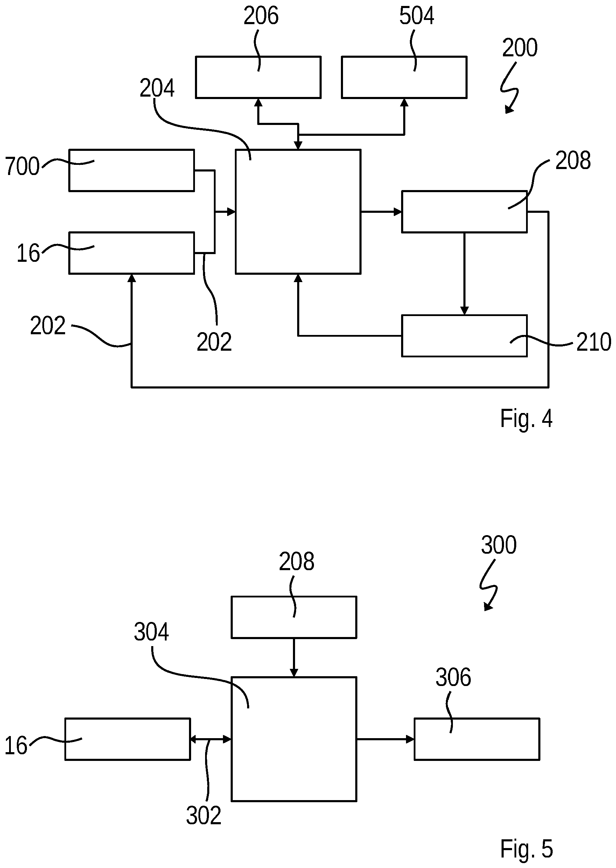

An embodiment of the appointment scheduling device 200 is shown in **Fig. 4****.** The appointment scheduling device 200 comprises an appointment determination unit 204, a treatment data storage unit 206, an appointment storage unit 208 and a user feedback unit 210. The appointment scheduling device 200 is configured to receive an appointment request and to determine, from the appointment request, at least one appointment.

The appointment request may comprise, in particular, information about the patient in question and a motivation of the appointment request. The motivation of the appointment request may, for example, be one of the following: first appointment requested by a new patient, one or more appointments required to fulfil a treatment regimen, one or more follow-up appointments. The appointment request may, for example, be entered into the appointment scheduling device 200 by means of the input/output device 700 or via another device of the medical operations support system 10 which has created an appointment request in the storage device 16 wherein the appointment request can be retrieved via the appointment scheduling data interface 202.

The appointment request is entered into the appointment determination unit 204 which request data on the requested treatment from the treatment data storage unit 206 if, for example, a medical treatment code 110 is included in the appointment request. Furthermore, the appointment determination unit 204 may request data on availability of personnel from a staff availability database 506. The appointment request together with, if applicable, data on the requested treatment and/or availability of personnel and/or further data constitutes input data for the appointment determination unit 204.

The appointment scheduling device 200 may, in some embodiments, be computer-implemented, for example by means of a computer, comprising at least one processor, volatile storage and/or non-volatile storage. To implement the appointment scheduling device 200, the implementation may comprise any of the following method steps:
Receive an appointment request data comprising, for example, one or more of the following data: patient identification data, patient medical record date, appointment type data, data describing the relationship of the appointment request with a treatment.

Determine, from the appointment request data, appointment requirement data, for example describing requirements for equipment, time, duration and/or personnel qualification. For example, certain appointments need to be carried out at specific times of day, require a medical professional qualified to draw blood or require exclusive use of equipment, for example a CT machine. These requirements are represented by appointment requirement data.

Search, in the appointment storage unit 208 and/or the staff availability database 506, for an appointment time slot meeting the appointment requirement data to create appointment proposal data.

Propose the appointment from the appointment proposal data to a user for confirmation, for example by means of the input/output device 700.

Receive confirmation of the proposed appointment from the user, for example by means of the input/output device 700, and store the proposed appointment in the appointment storage unit 208.

Receive a refusal of the proposed appointment from the user, for example by means of the input/output device 700, and continue with the search step to propose another appointment.

The appointment determination unit 204 may comprise a recurrent neural network (RNN) with reinforcement learning to suggest appointment dates and times to be stored in the appointment storage unit 208 and which may also be transmitted via the appointment scheduling data interface 202 to the data storage device 16. The user feedback device 210 may be used by patients seeking an appointment and receiving the suggested appointment dates and times to give feedback on the quality of the appointments suggested by the appointment determination unit 204. The feedback received by the appointment determination unit 204 is used to train the RNN by reinforcement learning.

### Alert Creation Device 300

The alert creation device 300 as shown in figure 5 comprises an alert dispatch unit 304 and a communication device 306. Furthermore, the alert dispatch unit 304 is connected to the data storage device 16 via the alert creation data interface 302 and to the appointment storage unit 208.

The communication device 306 comprises means for sending a message, in particular a push notification, via at least one communication means. The communication means may , for example, comprise an interface to the Internet, to a mobile phone network allowing the communication device 306 to send SMS or interfaces to chat networks like WhatsApp, Signal or Facebook messenger.

The alert dispatch unit 304 may be configured to retrieve appointment data from the appointment storage unit 208. In one step, the alert dispatch unit 304 analyses which of the patient is referenced in the appointment data has an upcoming appointment which is less than a predetermined time in the future. The upcoming appointment may, for example, be in less than two days. The alert dispatch unit 304 then obtains patient contact and preference data from the data storage device 16 to determine how to send a reminder to the patient about the upcoming appointment.

The alert dispatch unit 304 may further be configured to retrieve push notification data from the data storage device 16. Such push notification data may define certain push notifications to be dispatched by the alert dispatch unit 304, in particular push notifications relating to upcoming appointments, required tests, special offers or other relevant information.

The alert dispatch unit 304 may comprise an RNN to determine when an alert is to be dispatched. The RNN may be trained with reinforcement learning based on, for example, no-show after a reminder was sent or explicit customer feedback. The RNN will thus, over time, learn which alerts are sent at which times so that the benefit, for example appointment schedule compliance, is maximised.

### Diagnosis and Treatment Prediction Device 400

Diagnosis and treatment prediction device 400 comprises a diagnosis and treatment determination unit 404 which is connected to data storage device 16 via diagnosis and treatment data interface 402. Diagnosis and treatment determination unit 404 is configured to receive patient data, for example medical history data, age, gender and/or medical test result data, and is further configured to analyse the patient data to suggest diagnoses and/or propose treatments.

To this end, the diagnosis and treatment determination unit 404 may comprise an AI unit that is trained on vast medical datasets, including patient records, medical literature and clinical guidelines, to suggest potential diagnosis and/or treatment options based on each patient's unique patient data. The AI unit may, in particular, be trained on previously recorded patient data that is associated with a confirmed medical diagnosis. The AI unit may, furthermore, be trained on previously recorded treatment regimes and their influence on the patient's health data.

The treatment determination unit 404 may be configured to propose multiple diagnoses and/or treatments, wherein each of the diagnoses and/or treatments is assigned a confidence score which is a measure of how confident the AI unit is in the precision of the diagnosis and/or the adequacy of the treatment proposed. The confidence score may, for example, be a number between 0 (lowest confidence) and 100 (near certainty), easily indicating to a human operator how reliable the determined diagnosis and/or treatment options are. The treatment determination unit 404 may thus provide improved identification of potential diagnoses and treatment options, reducing the likelihood of misdiagnosis and incorrect treatments. Furthermore, the AI real-time analysis of patient data enables quicker clinical decision-making and timely interventions according to each patient's unique characteristics.

Furthermore, AI units may be more apt than human healthcare professionals to identify potential diagnoses and/or treatments for challenging or rare conditions, which would lead to better clinical outcomes. In some embodiments, care is taken to provide training data to the AI unit which is cleaned of certain biases, for example biases relating to race or gender.

The AI unit may be configured to be fed back to the diagnosis and/or treatment decision made by the healthcare professional such that predictions and recommendations of the AI unit may be continually refined and improved, becoming even more accurate and effective over time.

The AI unit may be configured to determine, beyond currently present diagnoses, risks for specific conditions, readmissions or complications from the medical patient data. Knowledge of such determined risks may enable healthcare professionals to proactively intervene and implement preventive measures to reduce said risks.

The diagnosis and treatment determination unit 404 may, in some embodiments, be supplemented by a medical information database 406. The medical information database 406 may contain additional medical information relating to diagnoses and treatments in a form that is intended for humans, for example as teaching or reference books. Furthermore, the medical information database 406 may contain supplementary information relating to details of diagnoses and treatments to medical professionals, for example when determining medication dosage and/or treatment regimes.

The diagnosis and treatment determination unit 404 may, in some embodiments, be connected to the input/output device 700. In these embodiments, the diagnosis and treatment determination unit 404 may be configured to implement an Al-powered chatbot or virtual assistant to gather patient information, assess symptoms and provide initial triage, helping to direct patients to the appropriate healthcare professional or service.

### Staff Management Device 500

Staff management device 500 comprises a staff allocation unit 504 connected to the data storage unit 16 via staff management data interface 502. The staff allocation unit 504 may comprise an AI module configured to analyze historical staff data, including attendance, performance, and patient feedback to predict staffing needs, peak performance times, and potential absences.

The staff management device 500 may be connected to appointment storage unit 208 by a communication link or by common data access, for example common access to data stored in the data storage device 16. The staff allocation unit 504 may provide data on available staff to the appointment storage unit 208 for the creation of appointments.

The purpose of staff management predictions as provided by staff allocation unit 504 in a clinic management system is to optimize resource allocation, enhance staff productivity, and improve overall clinic operations. By predicting staff availability, attendance, vacations, and sickness, the system can help clinic managers allocate human resources more effectively, ensuring that the clinic is adequately staffed at all times. Staff management predictions can identify the most productive hours for each employee, enabling clinic managers to schedule tasks and appointments during these optimal timeframes, ultimately increasing staff productivity and improving patient care.

Furthermore, by accurately predicting staffing needs and identifying potential bottlenecks, staff allocation unit 504 can help clinics avoid excessive overtime and staff burnout, promoting a healthier and more sustainable work environment. Effective staff management and optimized scheduling can lead to increased job satisfaction among employees, resulting in higher staff retention rates and reduced turnover costs.

Staff management predictions provide clinic managers with valuable insights into employee performance, allowing them to make informed decisions about hiring, promotions, and resource allocation. Optimized staff management can help clinics reduce labor costs by minimizing overtime, avoiding overstaffing, and ensuring that resources are allocated efficiently. Al-driven staff management predictions can adjust to changes in staff availability, patient demand, and other factors in real-time, ensuring that the scheduling process remains efficient and responsive to changing circumstances.

### Warehouse Management Device 600

Warehouse management device 600 comprises a warehouse prediction unit 604 connected to the data storage device 16 by means of the warehouse management data interface 602. The warehouse prediction unit 604 is connected to a warehouse database 606 in which current stock levels are recorded.

Warehouse prediction unit 604 may comprise an AI module configured to analyze historical data on product usage, stock levels, and supplier performance to optimize inventory management, identify the best suppliers, and negotiate the best prices for products. In some embodiments, warehouse prediction unit may be connected to input/output device 700 to visualize the AI module's predictions and recommendations, enabling clinic owners to make data-driven decisions about inventory management and supplier relationships. The purpose of warehouse management predictions in a clinic management system is to optimize inventory management, reduce costs, and ensure the timely availability of medical supplies and equipment.

By predicting product demand, stock levels, and reordering needs, warehouse management predictions help clinics maintain optimal inventory levels, ensuring that essential supplies and equipment are always available when needed. Accurate demand forecasting can help prevent stockouts, which could lead to delays in patient care, as well as overstocking, which can result in increased inventory holding costs and waste. Warehouse management predictions can help clinics identify the best time to order products, taking into account factors such as lead times, supplier reliability, and seasonal fluctuations, ensuring that supplies are replenished in a timely manner.

Optimized inventory management can lead to cost savings by reducing the need for emergency orders, minimizing inventory holding costs, and preventing waste due to expired or obsolete products. Warehouse management predictions can help clinics identify the best suppliers in terms of pricing, product quality, and delivery performance, enabling more informed decision-making and better supplier relationships. By providing insights into warehouse operations, staff productivity, and inventory levels, warehouse management predictions can help clinic managers allocate resources more efficiently, ensuring that warehouse operations run smoothly and cost-effectively. Warehouse management predictions give clinic managers access to valuable data that can be used to inform decisions about inventory management, supplier selection, and resource allocation.

### Input/Output Device 700

Input/Output device 700 may comprise an input means, a display means and none or more other human interface devices such as a mouse, a keyboard, a touchscreen, an audio interface such as a speaker or headphone. In some embodiments, the input/output device 700 is connected to one or more AI chatbots to facilitate human interaction with any of the invoice creation device 100, the appointment scheduling device 200, the alert creation device 300, the diagnosis and treatment device 400, the staff management device 500 and/or the warehouse management device 600. Output from the AI chatbot may, for example, be displayed by the input/output device 700 and input, for example text or audio input, may be provided to the AI chatbot.

In some embodiments, the input/output device 700 is configured as an API or a network interface, such as a webpage, and accessible from a computer such as a personal computer.

In some embodiments, the invoice creation module 100 may comprise a text standardization module configured to receive a medical text, for example a text from the medical note 104, and to convert the text of the medical note 104 into a standardized text according to a set of text formation rules and/or by means of an AI module.

The conversion may, for example, comprise steps to:
- Remove abbreviations and shorthand
- Correct grammar
- Convert single sentences into multiple sentences, in particular to separate multiple aspects convoluted in the sentence, in particular if these aspects are independent of each other
- Reword inacceptable phrasing, for example phrases that are disallowed by a medical care provider
- Highlight contradictory aspects of the text, for example incompatible combinations of diagnoses, medications and/or treatments, wherein incompatible may, in particular, mean harmful to patient health
- Replace mistakable and/or unclear wordings with more precise ones

The text standardization module may comprise a set of rules for the conversion and/or may be implemented and/or trained in the AI module.

While the medical operations support system 10 is shown in the embodiments to have a centralized storage device configured to store and retrieve different kinds of data, in particular configured as a database, in particular a relational database, each of the devices of the system may have its proper storage unit for storing data, for example local or operational data. Such proper storage units may be configured to allow access to the data stored therein to other devices of the system.

Optional features of the invention are designated with "may". Accordingly, there are also further embodiments and/or embodiments of the invention which additionally or alternatively have the respective feature or features.

From the presently disclosed combinations of features, isolated features can also be picked out, if necessary, and used in combination with other features to delimit the subject-matter of the claim, while removing the structural and/or functional relationship that may exist between the features.

### References

- 10: medical operations support system
- 16: data storage device
- 100: invoice creation device
- 102: invoice creation data interface
- 104: medical note
- 106: medical code determination unit
- 108: medical diagnosis code
- 110: medical procedure code
- 112: invoice coding unit
- 114: encoder module
- 116: hidden representation
- 118: decoder module
- 120: code interdependency repository
- 122: encoder context data
- 124: decoder context data
- 200: appointment scheduling device
- 202: appointment scheduling data interface
- 204: appointment determination unit
- 206: treatment data storage unit
- 208: appointment storage unit
- 210: user feedback unit
- 300: alert creation device
- 302: alert creation data interface
- 304: alert dispatch unit
- 306: communication device
- 400: diagnosis and treatment device
- 402: diagnosis and treatment data interface
- 404: diagnosis and treatment determination device
- 406: medical information database
- 500: staff management device
- 502: staff management data interface
- 504: staff management
- 506: staff availability database
- 600: warehouse management device
- 602: warehouse management data interface
- 604: warehouse prediction unit
- 606: warehouse database
- 700: input/output device
- 702: input/output data interface
- 800: medical device
- 802: medical data interface

## Claims

1. Medical operations support system (10), comprising two or more of the following devices:
a patient interaction device (700),
a patient data storage device (16) for storing patient data, configured to store and/or retrieve data upon request from another device of the medical operations support system (10),
a diagnosis and treatment device (400),
an invoice creation device (100),
an appointment scheduling device (200),
an alert creation device (300),
a staff management device (500),
a warehouse management device (600).

2. Medical operations support system according to claim 1, comprising the invoice creation device (100), **characterized in that** the invoice creation device (100) comprises a medical code determination unit (106) configured to determine one or more medical diagnosis codes (108) and/or medical procedure codes (110) from one or more medical notes (104).

3. Medical operations support system according to claim 2, **characterized in that** the medical code determination unit (106) comprises an AI module comprising an encoder module (114) configured to convert medical notes (104) to a hidden representation (116) and a decoder module (118) for converting the hidden representation (116) to a medical diagnosis code (108) and/or a medical procedure code (110).

4. Medical operations support system according to claim 1, **characterized in that** the invoice creation device (100) comprises a code interdependency repository (120) configured to guide the encoder module (114) and/or the decoder module (118) by providing information about which medical diagnosis codes (108) and/or medical procedure codes (110) may coexist and/or which medical diagnosis codes (108) and/or medical procedure codes (110) exclude each other.

5. Medical operations support system according to any of the previous claims, **characterized in that** any of the diagnosis and treatment device (400), the invoice creation device (100), the appointment scheduling device (200), the alert creation device (300), the staff management device (500) and the warehouse management device (600) may comprise its proper AI module or make use of a central AI module.

6. Medical operations support system according to any of the previous claims, comprising the invoice creation device (100), **characterized in that** the invoice creation device (100) comprises a text standardization module configured to receive a text of a medical note (104) and to convert the text of the medical note (104) into a standardized text, in particular according to a set of text formation rules and/or by means of an AI module.

7. Computer readable medium storing computer readable instructions that when executed by one or more computers cause the computers to form a medical operations support system (10) according to any of the previous claims or one or more of the patient interaction device (700), the patient data storage device (16), the diagnosis and treatment device (400), the invoice creation device (100), the appointment scheduling device (200), the alert creation device (300), the staff management device (500) and/or the warehouse management device (600).
